# EUROPEAN PATENT APPLICATION

(11) **EP 2 884 275 A1**
(43) Date of publication of application: **17.06.2015**
(21) Application number: 13817263.0
(22) Date of filing: 09.07.2013
(51) Int. Cl.: G01N 33/15, A61K 31/343, A61K 31/381, A61K 31/4045, A61K 45/00, A61P 7/12, A61P 43/00, C12Q 1/02, G01N 33/50, C12N 15/09

(54) **PHARMACEUTICAL COMPOSITION FOR TREATING OR PREVENTING STRESS URINARY INCONTINENCE OR MIXED INCONTINENCE, AND METHOD FOR SCREENING COMPOUNDS TO BE INCLUDED IN SAID PHARMACEUTICAL COMPOSITION**

(30) Priority: 10.07.2012 JP 2012154319
(71) Applicant: Astellas Pharma Inc., Chuo-ku Tokyo 103-8411 (JP)
(72) Inventor: WATANABE, Toru, Tokyo 103-8411 (JP); KAWABATA, Keiko, Tokyo 103-8411 (JP); WATANABE, Yuka, Tokyo 103-8411 (JP); YUYAMA, Hironori, Tokyo 103-8411 (JP); MASUDA, Noriyuki, Tokyo 103-8411 (JP); BURGARD, Edward C., Chapel Hill, North Carolina 27516 (US); THOR, Karl Bruce, Cary, North Carolina 27519 (US)
(74) Representative: Hoffmann Eitle
(86) International application number: PCT/JP2013/068781
(87) International publication number: WO 2014/010603

(57) **Abstract**

[Problem]

To provide a pharmaceutical composition for treating or preventing urinary incontinence on the basis of a novel mechanism of action, and a method for screening a compound to be comprised in the pharmaceutical composition as an active ingredient.

[Means for Solution]

The present invention relates to a pharmaceutical composition for treating or preventing stress urinary incontinence or mixed urinary incontinence, comprising a compound having a melatonin receptor activation action as an active ingredient. Examples of the active ingredient include melatonin, ramelteon, agomelatine, tasimelteon, TIK-301, and the like. Further, the present invention relates to a method for screening a compound to be comprised in a pharmaceutical composition for treating or preventing stress urinary incontinence or mixed urinary incontinence as an active ingredient, consisting of a step of measuring a melatonin receptor activation, a step of measuring the contraction of the isolated urethra, a step of measuring the urethral pressure, and the like.

## Description

### Technical Field

In a first aspect, the present invention relates to a screening method and a screening tool for a compound comprised in a pharmaceutical composition for treating or preventing stress urinary incontinence or mixed urinary incontinence. In a second aspect, the present invention relates to a pharmaceutical composition for treating or preventing stress urinary incontinence or mixed urinary incontinence, or the like.

### Background Art

Urinary incontinence is a condition in which there is involuntary leakage of urine is involved and recognized objectively and these become social or hygienic problems (Journal of Clinical Pharmacy and Therapeutics, 25, 251-263 (2000)). As the typical examples of urinary incontinence, urge urinary incontinence, stress urinary incontinence, functional urinary incontinence, reflex urinary incontinence, overflow urinary incontinence, genuine urinary incontinence, mixed urinary incontinence in which these types of urinary incontinence are mixed, and the like are known.

The most common type of urinary incontinence is stress urinary incontinence, and it has been reported that 50% of women having an onset of urinary incontinence have stress urinary incontinence (International Urogynecology Journal, 11 (5), 301-319 (2000)). Stress urinary incontinence refers to a disease in which with an increase in abdominal pressure such as coughing, sneezing, and exercise, or the like, urine leaks out involuntarily although the bladder is not contracted. The causes of stress urinary incontinence can be largely divided into two types. One is the bladder neck/urethra hypermobility, in which the transmission of abdominal pressure to the urethra fails due to bladder neck ptosis, based on the pelvic floor muscle relaxation, and thus, only the intravesical pressure increases and the urine leaks during an increase in the abdominal pressure. The other is that the reduction of a sphincter muscle function due to intrinsic sphincter deficiency causes urine leakage when the abdominal pressure rises. There is a high possibility that the onset of stress urinary incontinence is involved with weakening of the pelvic floor muscles due to aging and childbirth, and deterioration of the urethral function. In particular, the trauma of the pelvis due to pregnancy or vaginal childbirth is known as a risk factor for the onset of persistent stress urinary incontinence, and it has been reported that the prevalence of stress urinary incontinence for five years from the first childbirth is about 30% (Neurourology and Urodynamics, 21(1), 2-29 (2002)).

Urge urinary incontinence is a disease in which urine leaks involuntarily immediately after a strong and irrepressible urge to urinate (urination urgency), which occurs suddenly. Mixed urinary incontinence is a state having concurrent onset of a plurality of types of urinary incontinence, such as urge urinary incontinence, stress urinary incontinence, functional urinary incontinence, reflex urinary incontinence, overflow urinary incontinence, and genuine urinary incontinence, and in most cases, there are onsets of urge urinary incontinence and stress urinary incontinence.

Urinary incontinence has a major impact on the quality of life (QOL). Concerns about its symptoms restrict the range of activities of patients, making the patients feel loneliness and social isolation.

As a therapeutic agent for stress urinary incontinence, duloxetine having a serotonin-norepinephrine reuptake inhibiting action (SNRI), nisoxetine having a selective norepinephrine reuptake inhibiting action (NRI), and the like have been reported (International Urogynecology Journal,14, 367-372 (2003) and American Journal of Physiology - Renal Physiology, 292 (2), 639-646 (2007)). Norepinephrine and serotonin excite Onuf's nucleus that is a nucleus of an origin of somatic nerves controlling the urethra specific striated muscles in the reflex through the somatic nerves by an increase in the rapid intravesical pressure recognized during coughing, sneezing, and exercise. The excitement of the Onuf's nucleus excites the pudendal nerve that is a somatic nerve, and contracts the urethral specific striated muscles.

Duloxetine has been reported to have effectiveness for stress urinary incontinence in clinical trials, but has side effects such as nausea, insomnia, dizziness, suicidal tendencies, and the like. Nisoxetine has been reported to increase the urethral pressure as well as alleviate urine leakage induced by sneezing.

In the neuroreflex of the autonomic nerves by stretch stimulus of the bladder in the urine storage phase, an α₁ adrenoceptor is present in the urethra and plays a role in maintaining the continence by causing urethral contraction. To date, it has been reported that a plurality of drugs having α₁ adrenoceptor agonistic actions have a strong urethral contraction action, and in clinical trials, a drug having an α₁ adrenoceptor agonistic action is effective against stress urinary incontinence (Journal of Clinical Pharmacy and Therapeutics, 25, 251-263 (2000), International Urogynecology Journal, 14, 367-372 (2003), Urology, 62(Sup 4A), 31-38 (2003), and BJU International, 93, 162-170 (2004)). However, it has been reported that the α₁ adrenergic receptor agonist has cardiovascular side effects such as an increased blood pressure and the like.

As described above, it is considered that as a drug treatment for stress urinary incontinence, it is effective to increase the urethral resistance so as to maintain continence when the intravesical pressure rises during the urine storage phase, and thus, drugs based on some mechanisms of action have been studied. However, due to the problems such as side effects, development of a therapeutic agent for stress urinary incontinence based on a novel mechanism of action has been strongly desired.

Melatonin is a hormone secreted by the pineal gland, which exhibits an inhibitory effect on the growth and the function of reproductive glands. Melatonin has an effect on the circadian rhythm of animals, and plays a role in tuning the reproductive function to the light cycle of an environment.

It has been reported that melatonin alleviates nocturia, but the mechanism of action thereof has not been elucidated (Non-Patent Document 1). Further, it has been reported that melatonin alleviates a reduction in bladder contraction force due to aging or the like (Non-Patent Documents 2 to 6). On the other hand, it has been reported that melatonin inhibits bladder contractions (Non-Patent Documents 1 and 7) and melatonin alleviates overactive bladder (Non-Patent Document 8). However, it has been not reported that melatonin inhibits stress urinary incontinence or melatonin is involved in the contraction of the urethra at all. In addition, examples of diseases that may be affected by melatonin include diseases and disorders of the lower urinary tract (for example, dysuria and urinary incontinence), but there is no disclosure of specific data supporting this (Patent Document 5).

As a receptor of melatonin, three subtypes, MT1 (Mel1a), MT2 (Mel1b), MT3 (Mel1c), are known (Non-Patent Documents 9 and 10). MT1 and MT2 are G protein-coupled receptors (GPCR), which are coupled to Gᵢ and Gq, but MT3 has a quinone reduction enzyme (QR2) with a melatonin binding site. The affinity of melatonin to the MT1 and MT2 receptors is high, but the affinity to the MT3 receptor is low (Non-Patent Document 10).

Ramelteon (TAK-375, (S)-N-[2-(1,6,7,8-tetrahydro-2H-indeno[5,4-b]furan-8-yl)ethyl]propionamide) is a compound that has been found as an MT1 and MT2 receptor agonist, and is used for the treatment of insomnia characterized by difficulty in falling asleep (Non-Patent Documents 11 and 12).

Agomelatine (N-[2-(7-methoxynaphth-1-yl)ethyl]acetamide) is a compound having both an MT1 and MT2 receptor agonistic activity and a 5-HT2C receptor antagonistic activity, and is used for the treatment of depression (Patent Document 2).

Tasimelteon ((-)-(trans)-N- {[2-(2,3-dihydrobenzofuran-4-yl)cyclopropan-1-yl]methyl}propanamide) is a compound that binds to an MT1 and MT2 receptor, and is currently in Phase III trials for the purpose of treating non-24-hour sleep-wake disorders (Patent Document 3).

TIK-301 (N-[(2R)-2-(6-chloro-5-methoxy-1H-indol-3-yl)propyl]acetamide) is a compound that has an MT1 and MT2 receptor agonistic activity, and is currently in clinical trials for the purpose of treating insomnia (Patent Document 4).

As the MT1 and/or MT2 receptor agonist(s), several agonists have been reported, in addition to the compounds above, which are all for the purpose of the treatment of central nervous system diseases such as sleep disorder and depression, or the like. The agonist(s) to be used for treating urological diseases, typically such as urinary incontinence have not been reported.

### Related Art

### Patent Document

[Patent Document 1] KR-A-10-2011-090372
[Patent Document 2] EP-A-447285
[Patent Document 3] WO 98/025606
[Patent Document 4] EP-A-281242
[Patent Document 5] WO 2008/069311

### Non-Patent Document

[Non-Patent Document 1] Journal of Urology, 171 (3), 1199-1202 (2004)
[Non-Patent Document 2] Journal of Urology, 177 (4), 1558-1561 (2007)
[Non-Patent Document 3] Journal of Pineal Research, 44 (4), 416-425 (2008)
[Non-Patent Document 4] American Journal of Physiology - Regulatory, Integrative, and Comparative Physiology, 293 (2), R793-803 (2007)
[Non-Patent Document 5] Journal of Pharmacy and Pharmacology, 56(3), 359-366 (2004)
[Non-Patent Document 6] Curr. Med. Chem. 17 (34), 4150-4165, (2010)
[Non-Patent Document 7] BJU International, 94 (9), 1373-1376 (2004)
[Non-Patent Document 8] Acta. Medica., 54 (2), 63-68 (2011)
[Non-Patent Document 9] Cell and Tissue Research, 309, 151-162 (2002)
[Non-Patent Document 10] Proceedings of the National Academy of Sciences of the United States of America, 91, 6133-6137 (1994)
[Non-Patent Document 11] Neuropharmacology, 48, 301-310 (2005)
[Non-Patent Document 12] Annals of Neurology, 54 (suppl 7), S46 (2003)

### Disclosure of Invention

### Problems to Be Solved by the Invention

An object of the present invention is to provide a drug based on a novel mechanism of action as well as a screening method and a screening tool for a compound comprised in said pharmaceutical composition, as an active ingredient, in the treatment or prevention of stress urinary incontinence or mixed urinary incontinence. In particular, an object of the present invention is to provide a pharmaceutical composition for treating or preventing stress urinary incontinence or mixed urinary incontinence, comprising a compound having a melatonin receptor activating action as an active ingredient, and a method for screening a compound comprised as an active ingredient in the pharmaceutical composition.

### Means for Solving the Problems

The present inventors have conducted expression analysis, such as in the urethra, for the purpose of investigating a drug for treating stress urinary incontinence based a novel mechanism of action, and as a result, they have found that a gene of an MT1 receptor, which is one of subtypes of melatonin receptors, is highly expressed in the urethra. Further, the present inventors have also found that a compound having a melatonin receptor activating action contracts the urethra. Incidentally, the present inventors have also found that an MT1 receptor activating action is required for the urethra contracting action. Further, the present inventors have established a method for screening a compound for treating or preventing stress urinary incontinence or mixed urinary incontinence, using the melatonin receptor activating action as an index, and have thus found a pharmaceutical composition for treating or preventing stress urinary incontinence or mixed urinary incontinence, comprising a compound having a melatonin receptor activating action as an active ingredient.

That is, the present invention relates to the following:
[1] A method for selecting a compound for treating or preventing stress urinary incontinence or mixed urinary incontinence, comprising evaluating a melatonin receptor activating action.
[2] A method for selecting a compound for treating or preventing stress urinary incontinence or mixed urinary incontinence, comprising:
   a step of bringing the compound into contact with a melatonin receptor expressing cell;
   a step of measuring a melatonin receptor activation; and
   a step of selecting a compound that activates a melatonin receptor.
[3] The method described in [2] above, further comprising a step of confirming that the selected compound has a therapeutic effect on stress urinary incontinence or mixed urinary incontinence.
[4] The method described in [2] above, further comprising:
   a step of bringing the selected compound into contact with the isolated urethra;
   a step of measuring the contraction of the isolated urethra; and
   a step of selecting a compound which contracts the isolated urethra.
[5] The method described in [3] above, wherein the step of confirming the therapeutic effect comprises:
   a step of administering the selected compound to a subject;
   a step of measuring the urethral pressure of the subject; and
   a step of selecting a compound increasing the urethral pressure.
[6] The method described in [3] above, wherein the step of confirming the therapeutic effect is a step of measuring the parameters of urinary incontinence before and after administering the selected compound to a subject.
[7] The method described in any one of [1] to [6] above, wherein the melatonin receptor is an MT1 receptor.
[8] A screening tool used in a method for selecting a compound for treating or preventing stress urinary incontinence or mixed urinary incontinence, comprising a melatonin receptor polypeptide or a melatonin receptor expressing cell.
[9] Use of a melatonin receptor polypeptide or a melatonin receptor expressing cell for the manufacture of a screening tool used in a method for selecting a compound for treating or preventing stress urinary incontinence or mixed urinary incontinence.
[10] Use of a melatonin receptor polypeptide or a melatonin receptor expressing cell as a screening tool used in a method for selecting a compound for treating or preventing stress urinary incontinence or mixed urinary incontinence.
[11] A pharmaceutical composition for treating or preventing stress urinary incontinence or mixed urinary incontinence, comprising a compound having a melatonin receptor activating action as an active ingredient.
[12] The pharmaceutical composition described in [11] above, wherein the melatonin receptor is an MT1 receptor.
[13] The pharmaceutical composition described in [11] or [12] above, wherein the compound having a melatonin receptor activating action is a compound selected from the group consisting of melatonin, ramelteon, agomelatine, tasimelteon, and TIK-301 and a pharmaceutically acceptable salt thereof.
[14] A method for treating or preventing stress urinary incontinence or mixed urinary incontinence, comprising administering an effective amount of a compound having a melatonin receptor activating action to a subject.
[15] The method described in [14] above, wherein the melatonin receptor is an MT1 receptor.
[16] The method described in [14] or [15] above, wherein the compound having a melatonin receptor activating action is a compound selected from the group consisting of melatonin, ramelteon, agomelatine, tasimelteon, and TIK-301 and a pharmaceutically acceptable salt thereof.
[17] Use of a compound having a melatonin receptor activating action for the manufacture of a pharmaceutical composition for treating or preventing stress urinary incontinence or mixed urinary incontinence.
[18] The use of the compound described in [17] above, wherein the melatonin receptor is an MT1 receptor.
[19] The use of the compound described in [17] or [18] above, wherein the compound having a melatonin receptor activating action is a compound selected from the group consisting of melatonin, ramelteon, agomelatine, tasimelteon, and TIK-301 and a pharmaceutically acceptable salt thereof.
[20] Use of a compound having a melatonin receptor activating action for the treatment of stress urinary incontinence or mixed urinary incontinence.
[21] The use of the compound described in [20] above, wherein the melatonin receptor is an MT1 receptor.
[22] The use of the compound described in [20] or [21] above, wherein the compound having a melatonin receptor activating action is a compound selected from melatonin, ramelteon, agomelatine, tasimelteon, and TIK-301 and a pharmaceutically acceptable salt thereof.
[23] A compound having a melatonin receptor activating action for the treatment of stress urinary incontinence or mixed urinary incontinence.
[24] The compound described in [23] above, wherein the melatonin receptor is an MT1 receptor.
[25] The compound described in [23] or [24] above, wherein the compound having a melatonin receptor activating action is a compound selected from the group consisting of melatonin, ramelteon, agomelatine, tasimelteon, and TIK-301 and a pharmaceutically acceptable salt thereof.

Further, the present invention relates to the following:
[25] The pharmaceutical composition described in [11] to [13] above, which is used in combination with duloxetine.
[26] The method described in [14] to [16] above, which is used in combination with duloxetine.
[27] The use described in [17] to [19] above, which is used in combination with duloxetine.

Meanwhile, the term "subject" is a human being or another animal in need of prevention or treatment thereof, and according to a certain embodiment, a human being in need of prevention or treatment thereof.

Furthermore, in one embodiment, the urinary incontinence is stress urinary incontinence, urge urinary incontinence, or mixed urinary incontinence, and in another embodiment, stress urinary incontinence or mixed urinary incontinence. Further, in still another embodiment, the urinary incontinence is stress urinary incontinence, and in even still another embodiment, mixed urinary incontinence.

In the present specification, stress urinary incontinence is a disease in which with an increase in abdominal pressure such as coughing, sneezing, exercise, and the like, urine leaks out involuntarily although the bladder is not contracted; urge urinary incontinence is a disease in which urine leaks involuntarily immediately after a strong and irrepressible urge to urinate (urination urgency), which occurs suddenly; and mixed urinary incontinence means a disease having an onset of at least one selected from the group consisting of urge urinary incontinence, functional urinary incontinence, reflex urinary incontinence, overflow urinary incontinence, and genuine urinary incontinence, in combination with stress urinary incontinence, and in one embodiment, mixed urinary incontinence is a disease having an onset of urge urinary incontinence and stress urinary incontinence.

### Effects of the Invention

The present invention is useful in providing to a pharmaceutical composition for treating or preventing stress urinary incontinence or mixed urinary incontinence, based on a novel mechanism of action; and a screening method and a screening tool for a compound comprised in the pharmaceutical composition as an active ingredient.

### Brief Description of Drawings

Fig. 1 shows expression level of MT1 receptor in a normal tissue of a rat. The vertical axis indicates the number of copies of a gene present in the reaction solution. In the figure, DRG represents dorsal root ganglion.
Fig. 2 shows melatonin receptor activating actions of melatonin and ramelteon in human MT1 receptor expressing cells. The vertical axis indicates a change in the fluorescent strength in response to a change in the intracellular Ca²⁺ concentration.
Fig. 3 shows the melatonin receptor activating actions of melatonin and ramelteon in human MT2 receptor expressing cells. The vertical axis indicates a change in the fluorescent strength in response to a change in the intracellular Ca²⁺ concentration.
Fig. 4 shows the melatonin receptor activating actions of agomelatine, tasimelteon, and TIK-301 in human MT1 receptor expressing cells. The vertical axis indicates a change in the fluorescent strength in response to a change in the intracellular Ca²⁺ concentration.
Fig. 5 shows the melatonin receptor activating actions of agomelatine, tasimelteon, and TIK-301 in human MT2 receptor expressing cells. The vertical axis indicates a change in the fluorescent strength in response to a change in the intracellular Ca²⁺ concentration.
Fig. 6 shows the urethra contracting actions of melatonin and ramelteon in the isolated urethra. The vertical axis indicates the contraction strength of the compound where the contraction strength by 10⁻⁵ M phenylephrine is set to 100%.
Fig. 7 shows the urethra contracting actions of agomelatine, tasimelteon, and TIK-301 in the isolated urethra. The vertical axis indicates the contraction strength of the compound where the contraction strength by 10⁻⁵ M phenylephrine is set to 100%.
Fig. 8 shows the urethral pressure increasing action of melatonin in rats, as measured using a microchip method. The vertical axis indicates a change in the urethral pressure between before and after the addition of the compound as Δ Elevation value.
Fig. 9 shows the urethral pressure increasing action of ramelteon in a rat, as measured using a microchip method. The vertical axis indicates a change in the urethral pressure between before and after the addition of the compound as Δ Elevation value.
Fig. 10 shows the urethral pressure increasing action of melatonin in a rat, as measured using a perfusion method. The vertical axis indicates a change in the urethral pressure between before and after the addition of the compound as Δ Elevation value.
Fig. 11 shows the urethral pressure increasing action of ramelteon in a rat, as measured using a perfusion method. The vertical axis indicates a change in the urethral pressure between before and after the addition of the compound as Δ Elevation value.
Fig. 12 shows the urethral pressure increasing action of agomelatine in a rat, as measured using a perfusion method. The vertical axis indicates a change in the urethral pressure between before and after the addition of the compound as Δ Elevation value.
Fig. 13 shows the urethral pressure increasing action of tasimelteon in a rat, as measured using a perfusion method. The vertical axis indicates a change in the urethral pressure between before and after the addition of the compound as Δ Elevation value.
Fig. 14 shows the urethral pressure increasing action of TIK-301 in a rat, as measured using a perfusion method. The vertical axis indicates a change in the urethral pressure between before and after the addition of the compound as Δ Elevation value.
Fig. 15 shows an effect of the addition of luzindole on the melatonin receptor activating action of ramelteon in a human MT1 receptor expressing cell. The vertical axis indicates the effect of the intracellular Ca²⁺ increasing action in the presence of the compound where the action by 10⁻⁶ M ramelteon is set to 100%.
Fig. 16 shows an effect of the addition of luzindole on the melatonin receptor activating action of ramelteon in a human MT2 receptor expressing cell. The vertical axis indicates the effect of the intracellular Ca²⁺ increasing action in the presence of the compound where the action by 10⁻⁶ M ramelteon is set to 100%.
Fig. 17 shows an effect of the addition of luzindole on the urethra contracting action of ramelteon in the isolated urethra. The vertical axis indicates the contraction strength in the presence of the compound where the action by 10⁻⁶ M ramelteon is set to 100%.
Fig. 18 shows the isolated uterus contracting action by melatonin. The vertical axis indicates the contraction strength of the compound where the contraction by 60 mM KCl is set to 100%.
Fig. 19 shows the isolated bladder contraction action and the isolated uterus contracting action by ramelteon. The vertical axis indicates, in a case of the isolated bladder, the contraction strength of the compound where the contraction by 10⁻⁶ M carbachol is set to 100%, and in a case of the isolated uterus, the contraction strength of the compound where the contraction by 60 mM KCl is set to 100%.
Fig. 20 shows the effect of ramelteon on the rhythmic contraction of the isolated uterus. The vertical axis indicates a frequency of rhythmic contractions of the uterus where the frequency before the treatment of the compound is set to 100%.
Fig. 21 shows the effect of melatonin on the rat mean blood pressure (MBP). The vertical axis indicates a change rate in the mean blood pressure.
Fig. 22 shows the effect of ramelteon on the rat mean blood pressure. The vertical axis indicates a change rate in the mean blood pressure.

Further, for the sake of convenience in the present specification, concentration of mol/L is represented by M.

### Embodiments for Carrying Out the Invention

Hereinbelow, the present invention will be described in more detail.

### 1. Screening Tool

Examples of the screening tool constituting the present invention include a polypeptide or an expression cell.

Examples of the polypeptide which can be used as a screening tool constituting the present invention include a polypeptide consisting of the same amino acid sequence as the MT1 and/or MT2 receptor(s) of mammals, and a polypeptide functionally equivalent thereto.

The functionally equivalent polypeptide is not particularly limited as long as it is activated by an MT1 and/or MT2 receptor agonist(s) and can transfer an activity to the intracellular signaling system, and examples of the functionally equivalent polypeptide include a polypeptide comprising the same amino acid sequence as the MT1 and/or MT2 receptor(s) of mammals, a polypeptide comprising an amino acid sequence having deletion, substitution, and/or insertion of 1 to 10 amino acids in the amino acid sequence of the MT1 and/or MT2 receptor(s) of mammals, and a polypeptide comprising an amino acid sequence having an identity of 90% or more with respect to the amino acid sequence of the MT1 and/or MT2 receptor(s) of mammals.

In addition, "identity" in the present specification means an identity obtained using parameters prepared as the defaults in searching with the NEEDLE program (J. Mol. Biol. 1970; 48: 443-453). The parameters are as follows.
Gap penalty = 10
Extend penalty = 0.5
Matrix = EBLOSUM62

Examples of the polypeptide comprising the amino acid sequence of the MT1 and/or MT2 receptor(s) of mammals further include a polypeptide having an addition of a suitable marker sequence or the like at the N terminal and/or C terminal of a polypeptide consisting of the amino acid sequence of the MT1 and/or MT2 receptor(s) of mammals. In the present invention, examples of the polypeptide consisting of the amino acid sequence of the MT1 and/or MT2 receptor(s) of mammals, which can be used as a screening tool, include MT1 and/or MT2 receptor(s) derived from humans, mice, and rats (Neuron, 13 (5), 1177-1185 (1994), Endocrinology, 137 (8), 3469-3477(1996), Proc. Natl. Acad. Sci. U.S.A., 92, 8734-8738 (1995), Biochem. Biophys. Res. Commun., 262 (3), 832-837 (1999)). In the present invention, MT1 and/or MT2 receptor(s) derived from humans are particularly preferred.

The MT1 and/or MT2 receptor expressing cell(s) which can be used as a screening tool constituting the present invention is not particularly limited as long as it expresses the polypeptide for a screening tool described above. The MT1 and/or MT2 receptor expressing cell(s) may be transformed cells in which a polypeptide of an MT1 and/or MT2 receptor(s) is artificially expressed by transformation with a foreign gene, or a natural cell or a cell line thereof in which the polypeptide of the MT1 and/or MT2 receptor(s) is expressed.

Furthermore, in one embodiment, the "MT1 and/or MT2 receptor(s)" are an MT1 receptor and an MT2 receptor, in another embodiment, an MT1 receptor, and in a still another embodiment, an MT2 receptor.

### 2. Method for Manufacturing Screening Tool

The screening tool of the present invention can be manufactured according to a known method (for example, Molecular Cloning-A Laboratory Manual, Cold Spring Harbor Laboratory, NY, 1989).

The MT1 and/or MT2 receptor expressing cell(s) which can be used in the screening tool of the present invention can be obtained by introducing a polynucleotide encoding a polypeptide which can be used in the screening tool of the present invention into a suitable vector to transform suitable host cells. Further, a polypeptide which can be used in the screening tool of the present invention can be obtained by being separated and produced from cultured transformed cells by a known method (for example, Molecular Cloning-A Laboratory Manual, Cold Spring Harbor Laboratory, NY, 1989).

The method for preparing a polynucleotide encoding a polypeptide which can be used in the screening tool of the present invention is not particularly limited, but can be prepared by using an RT-PCR method using a pair of primers having each of a human MT1 receptor gene (GenBank Access No.: NM_005958.3) and a human MT2 receptor gene (GenBank Access No.: NM_005959.3) between desired sequences, a chemical synthesis method, or the like.

The host cells which can be used to prepare transformed cells are not particularly limited as long as they can be expressed to allow a melatonin receptor to function, and examples thereof include a human embryonic kidney derived HEK293 cell (J. Gen. Virol., 36 (1), 59-74 (1977)), a Chinese hamster ovary (CHO) cell (Proc. Natl. Acad. Sci. USA, 60, 1275-1281 (1968)), and a COS cell that is a monkey cell (Cell, 23, 175-182 (1981)).

The expression vector which can be used to prepare the transformed cell is not particularly limited as long as it can express a melatonin receptor, and can be appropriately selected according to the kind of host cells used. As an expression vector, a vector containing a promoter positioned upstream of the polynucleotide to be expressed, an RNA splicing site, a polyadenylation site, and a transcription termination sequence may be generally used, and the vector may further contain a replication origin, if necessary. Examples of the expression vector which can be used include pCDNA3.1 (Invitrogen Inc.).

The method for introducing the expression vector into a host cell is not particularly limited, but examples thereof include a DEAE-dextran method (Nucleic. Acids. Res., 11, 1295-1308 (1983)), a calcium phosphate-DNA co-precipitation method (Virology, 52, 456-457 (1973)), and a method using a commercially available transfection reagent (for example, Lipofectamine (registered trademark) (Invitrogen Inc.), or FUGENE (registered trademark) (Roche Diagnostics)).

The transformed cell can be cultured in accordance with a conventional method, and as a medium, various media commonly used in a host cell employed can be appropriately selected. For example, Dulbecco's modified Eagle's Medium (DMEM) (Invitrogen Inc.), MEMα (Invitrogen Inc.), RPMI-1640 (Invitrogen Inc.), or the like may be used. Further, the medium may be supplemented with a serum component such as fetal bovine serum (FBS), if necessary. Further, a drug such as Neomycin, Hygromycin, and Zeocin may be appropriately added for selection of genes.

### 3. Screening Method comprising Measuring Melatonin Receptor Activation

The "melatonin receptor activating" action means any of actions in an initial step for causing functional expression by a ligand-binding receptor by a compound directly or indirectly binding to a ligand binding site of a melatonin receptor to perform an action. Examples thereof include an agonistic action or partial agonistic action for a melatonin receptor and a melatonin receptor activating action by an allosteric effect, but are not limited thereto.

Examples of the method for "measuring the melatonin receptor activation" include measurement of the melatonin receptor activation by bringing a compound into contact with a melatonin receptor expressing cell.

The "melatonin receptor expressing cell" may be a transformed cell which is transformed to an expression vector comprising a polynucleotide encoding any protein of MT1 and/or MT2 receptor(s) and expresses any one of MT1 and/or MT2 receptor(s), or may be a natural cell or a cell line thereof, which expresses an MT1 and/or MT2 receptor polypeptide(s).

Examples of the method for preparing a transformed cell include the methods as described above in the section of "2. Method for Preparing Screening Tool".

The method for bringing the melatonin receptor expressing cell into contact with the compound is not particularly limited, but, for example, the transformed cells may be seeded in a culture plate to a desired cell density and a compound solution adjusted to a desired final concentration may be added thereto.

As an index of the activation of melatonin receptors, it is possible to measure factors such as cAMP downstream of Gᵢ-coupled GPCR. The factor as an index is not particularly limited, but by using G₁₅, G₁₆, or chimeric G protein of Gq and Gᵢ as a coupled G protein, for example, the intracellular concentrations of calcium ions (Ca²⁺), inositol-3-phosphoric acid (IP3), inositol-1-phosphoric acid (IP1), and the like can be measured. In another embodiment, a receptor affinity calculated by a ligand binding assay method can be used as an index.

The intracellular concentrations of cAMP, Ca²⁺, IP3, IP1, and the like can be measured using a labeling agent. As the labeling agent, a radioisotope, an enzyme, a fluorescent substance, a luminescent substance, or the like is used. As the radioisotope, for example, [³²P] or the like is used. As the enzyme, an enzyme which is stable and has high specific activity is preferred, and as an example thereof, alkaline phosphatase, peroxidase, malate dehydrogenase, and the like are mentioned. Examples of the fluorescent substance include fluorescamine and fluorescein isothiocyanate (FITC). Examples of the light emitting material include luminol, a luminol derivative, luciferin, and lucigenin.

Preferred examples of the measuring method include a method of measuring an intracellular Ca²⁺ concentration or an intracellular IP1 concentration. The intracellular Ca²⁺ concentration can be measured by introducing a Ca²⁺-sensitive fluorescent dye such as Fluo4 and Fluo4-AM (Dojindo Co.) into a cultured cell, and using an FLIPR (registered trademark) system (Molecular Device Inc.). The intracellular IP1 concentration can be measured by adding a reagent equipped with an IP-One measuring kit (Cisbio Inc.) to a cell lysate and using time-resolved fluorescence in a plate reader.

In the presence of the compound, if there is a change in the index of the melatonin receptor activation as compared to the absence of the compound, the compound can be determined to be a compound which activates the melatonin receptor. In another embodiment, if the change in the index of the melatonin receptor activation varies depending on the concentration of the compound, the compound can be determined as a compound which activates the melatonin receptor. Specifically, a compound having, in one embodiment, an EC₅₀ value less than 10 µM, in another embodiment, an EC₅₀ value less than 1 µM, in still another embodiment, an EC₅₀ value less than 100 nM, or in even still another embodiment, an EC₅₀ value less than 10 nM may be selected as a compound which activates the melatonin receptor.

Confirmation of the effectiveness of the compound obtained by the screening method comprising a step of measuring the melatonin receptor activation in the present invention for the treatment or prevention of stress urinary incontinence or mixed urinary incontinence can be carried out by using a method known to a person skilled in the art or a modified method thereof, for example, the method described in the section of "5. Screening Method Using Isolated Urethra", "6. Screening Method for Measuring Urethral Pressure", or "7. Method for Measuring Parameters of Urinary Incontinence Using Animals". More specifically, examples of the methods include the methods described in Examples 3 and 4.

### 4. Compound Having Melatonin Receptor Activating Action

Examples of the compound to be tested by the method of the present invention include candidate compounds for pharmaceuticals or animal drugs. Examples of the "compound having a melatonin receptor activating action" include a compound found to activate a melatonin receptor through a step of bringing a melatonin receptor expressing cell into contact with the compound as well as a synthetic compound or natural compound known to have a melatonin receptor activating action, and in a case of the synthetic compound, include various well-known compounds (including peptides) registered in chemical files, compounds obtained by combinatorial chemistry techniques (Tetrahedron, 51, 8135-8137 (1995)) or the like, and a group of random peptides prepared by applying a phage display method (J. Mol. Biol., 222, 301-310, (1991)) or the like. In addition, in a case of the natural compound, for example, components derived from microorganisms, plants, marine organisms, or animals (for example, culture supernatants and tissue extracts) can be used.

The melatonin receptor is known to have three kinds of subtypes of MT1, MT2, and MT3. In one embodiment, the compound having a melatonin receptor activating action in the present invention is a compound having an MT1 receptor agonistic action, an MT2 receptor agonistic action, or an MT1 and MT2 receptor agonistic action.

In one embodiment, the compound having a melatonin receptor activating action in the present invention is melatonin, ramelteon, agomelatine, tasimelteon, or TIK-301, or a pharmaceutically acceptable salt thereof.

Melatonin or a pharmaceutically acceptable salt thereof is available by purchase from Sigma, or the like. Ramelteon or a pharmaceutically acceptable salt thereof is available by the production process described in the pamphlet of WO 97/32871 or a production process equivalent thereto. Agomelatine or a pharmaceutically acceptable salt thereof is available by the production process described in the EP-A-447285 or a production process equivalent thereto. Tasimelteon or a pharmaceutically acceptable salt thereof is available by the production process described in the pamphlet of WO 98/25606 or a production process equivalent thereto. TIK-301 or a pharmaceutically acceptable salt thereof is available by the production process described in the EP-A-281242 or a production process equivalent thereto.

However, the compound to be tested by the method of the present invention is not limited to these compounds, and examples thereof include compounds having an MT1 receptor agonistic action, an MT2 receptor agonistic action, and an MT1 and MT2 receptor agonistic action. Further, the compound having an MT1 receptor agonistic action, an MT2 receptor agonistic action, and an MT1 and MT2 receptor agonistic action can be easily obtained by using a melatonin receptor expressing cell or the like.

The compound to be tested by the method of the present invention may exist in the form of tautomers or geometrical isomers depending on the kind of substituents. In the present specification, the test compound may be described in only one form of isomer, but the compound to be tested by the method of the present invention includes such an isomer, isolated forms of the isomers, or a mixture thereof.

In addition, the compound to be tested by the method of the present invention may have asymmetric carbon atoms or axial asymmetry in some cases, and correspondingly, it may exist in the form of optical isomers. The compound to be tested by the method of the present invention includes an isolated form of the optical isomers or a mixture thereof.

For the compound to be tested by the method of the present invention, the pharmaceutically acceptable salt may form an acid addition salt or a salt with a base depending on the kind of substituents. Specific examples thereof include acid addition salts with inorganic acids such as hydrochloric acid, hydrobromic acid, hydroiodic acid, sulfuric acid, nitric acid, and phosphoric acid, and with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, p-toluenesulfonic acid, aspartic acid, and glutamic acid, and salts with inorganic bases such as sodium, potassium, magnesium, calcium, and aluminum, or organic bases such as methylamine, ethylamine, ethanolamine, lysine, and ornithine, salts with various amino acids such as acetylleucine, and amino acid derivatives, as well as ammonium salts.

The compound to be tested by the method of the present invention further includes various hydrates or solvates, and polymorphic crystal substances of the compound. In addition, the compound to be tested by the method of the present invention also includes compounds labeled with various radioactive or non-radioactive isotopes.

The pharmaceutical composition of the present invention can be prepared using excipients that are usually used in the art, that is, excipients for pharmaceutical preparation, carriers for pharmaceutical preparation, and the like according to the methods usually used. Administration can be accomplished either by oral administration via tablets, pills, capsules, granules, powders, solutions, or the like, or by parenteral administration with injections such as intraarticular, intravenous, and intramuscular injections, suppositories, ophthalmic solutions, eye ointments, transdermal liquid preparations, ointments, transdermal patches, transmucosal liquid preparations, transmucosal patches, inhalers, or the like.

As the solid composition for oral administration according to the present invention, tablets, powders, granules, or the like are used. In such a solid composition, one or more active ingredient(s) are mixed with at least one inactive excipient. In a conventional method, the composition may contain inactive additives, such as a lubricant, a disintegrating agent, a stabilizer, or a solubilization assisting agent. The tablets or pills may be coated with sugar or a film of a gastric or enteric coating substance, if necessary.

The liquid composition for oral administration contains pharmaceutically acceptable emulsions, solutions, suspensions, syrups, elixirs, or the like, and also contains generally used inert diluents, for example, purified water or ethanol. In addition to the inert diluent, the liquid composition may also contain auxiliary agents, such as a solubilization assisting agent, a moistening agent, and a suspending agent, sweeteners, flavors, aromatics, or antiseptics.

The injections for parenteral administration include sterile aqueous or non-aqueous solution preparations, suspensions and emulsions. The aqueous solution or suspension includes, for example, distilled water for injection and physiological saline. Examples of the non-aqueous solution or suspension include alcohols such as ethanol. Such a composition may further contain a tonicity agent, an antiseptic, a moistening agent, an emulsifying agent, a dispersing agent, a stabilizing agent, or a solubilizing aid. These are sterilized, for example, by filtration through a bacteria retaining filter, blending with a bactericide, or irradiation. In addition, these can also be used by preparing a sterile solid composition, and dissolving or suspending it in sterile water or a sterile solvent for injection prior to its use.

Examples of the agent for external use include ointments, plasters, creams, jellies, patches, sprays, lotions, eye drops, eye ointments, and the like. The agents contain generally used ointment bases, lotion bases, aqueous or non-aqueous liquid preparations, suspensions, emulsions, or the like.

As the transmucosal agents such as an inhaler and a transnasal agent, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For the administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device, and the like. A dry powder inhaler or the like may be for single or multiple administration use, and a dry powder or a powder-containing capsule may be used. Alternatively, this may be in a form such as a pressurized aerosol spray which uses an appropriate propellant agent, for example, a suitable gas such as a chlorofluoroalkane, a hydrofluoroalkane, and carbon dioxide, or other forms.

The drug used in the present invention is administered to a patient with stress urinary incontinence or mixed urinary incontinence. The dose is appropriately decided in response to an individual case by taking the administration method, the symptoms, the age, the gender, and the like into consideration.

Furthermore, even if it is not clear that the compound has a melatonin receptor activating action, the above-described method for determining the compound as a compound activating the melatonin receptor can be used for determining whether the compound has a melatonin receptor activating action or not.

By using a compound having a melatonin receptor antagonistic action, it can be confirmed that the action of contracting the isolated urethra of the compound is an action based on the melatonin receptor activating action. For example, when the action of contracting the isolated urethra of the compound is weakened by a compound having a melatonin receptor antagonistic action, it can be said that the compound is a compound having a melatonin receptor activating action.

As the compound having a melatonin receptor antagonistic action, known synthesized compounds or natural substances may be used without particular limitation. Here, the melatonin receptor antagonist may be any one of an MT1 receptor antagonist, an MT2 receptor antagonist, or an MT1 and MT2 receptor antagonist. Preferred examples of the melatonin receptor antagonist include luzindole (N-[2-[2-(phenylmethyl)-1H-indol-3-yl]ethyl]acetamide) (J. Pharmacol. Exp. Ther. 246, 902-910 (1988)). Luzindole is easily available by purchase from Nelson Research, Inc.

The method for bringing the compound having a melatonin receptor antagonistic action into contact with the isolated urethra is not particularly limited, but, for example, a compound adjusted to have a desired final concentration can be added to a buffering solution having the isolated urethra therein.

The compound having a melatonin receptor antagonistic action is preferably brought into contact with the isolated urethra at a timing which allows the compound to sufficiently exhibit the action. The timing for bringing into contact is not particularly limited, but, for example, a case where the compound having a melatonin receptor antagonistic action is added a certain time earlier than the time for the addition of the compound into a buffering solution having the isolated urethra therein, or at the same time as the addition of the compound may be mentioned.

The method for administering the compound having a melatonin receptor antagonistic action to an animal is not particularly limited as long as the compound is administered to the animal so as that a sufficient amount of the compound is delivered to target tissues (for example, urethra, bladder, uterus, and blood vessel). As the dosage form, any administration method can be selected, as long as it is suitable for the compound.

The compound having a melatonin receptor antagonistic action is preferably administered to an animal at a timing that allows the compound to sufficiently exhibit the action. The timing for administration is not particularly limited, but examples thereof include a timing where the concentration of the compound having a melatonin receptor antagonistic action in the blood increases such that the compound can exhibit an antagonistic action in the target tissue. Specifically, the compound having a melatonin receptor antagonistic action may be added a certain time earlier than the administration of the compound to an animal or at the same time as the administration of the compound.

### 5. Screening Method Using Isolated Urethra

The "isolated urethra" means the urethra tissue isolated from the body in the state of maintaining the contractility and relaxability from the mammals as described later in the section of "6. Screening Method for Measuring Urethral Pressure". The urethra tissue isolated from the body is rapidly preserved in a buffering solution and used for an experiment for measuring the contractility. The buffering solution for the isolated urethra is not particularly limited, but examples thereof include a Krebs-Henseleit solution.

The method of bringing the compound into contact with the isolated urethra is not particularly limited, but, for example, a compound adjusted to a desired final concentration may be added to a buffering solution for the isolated urethra.

As a method for measuring the contraction of the isolated urethra, a conventional method can be used. Specifically, the isolated urethra is incised vertically and then made into strip-shaped specimens. The specimens are suspended in an equilibrated buffering solution at a constant temperature. The contraction response by the compound is measured using a pickup pressure transducer connected to a modified pressure amplifier and recorded in a pen recorder. The specimens may be suspended in any of the circular muscle direction or the longitudinal muscle direction. Preferably, the specimens are suspended in the circular muscle direction. In a case of considering the action with an exclusion of the involvement of the urethral sphincter, the sphincter portion can be resected from the strip-shaped specimens to prepare urethral smooth muscle specimens, and then the specimens may be suspended.

In the present invention, a step of selecting a compound that specifically contracts the isolated urethra can be further comprised. Here, the expression of "specifically" contracting means that the compound contracts the isolated urethra whereas it does not contract other tissues, for example, isolated bladder, isolated uterus, or the like, or the degree of contraction of other tissues is smaller, relative to the contraction of the isolated urethra.

The isolated bladder or the isolated uterus means a bladder tissue or a uterus tissue which is isolated from the body while maintaining contractility relaxability from the above-described mammals. For an experiment for measuring the contractility of the isolated bladder or the isolated uterus, a method equivalent to the above-described experiment for measuring the isolated urethra can be used.

In one embodiment, the compound which contracts the isolated urethra is a compound in which the isolated urethra contracting action shown in Example 3 as described later exhibits an action of 100% or more.

### 6. Screening Method for Measuring Urethral Pressure

As a subject to which the compound is administered, mammals are preferred. In one embodiment, the mammals are humans, monkeys, rats, mice, hamsters, guinea pigs, dogs, cats, rabbits, pigs, sheep, goats, horses, cattle, or the like. In another embodiment, the mammals are humans, monkeys, dogs, rats, mice, hamsters, or the like, in still another embodiment, humans, in even still another embodiment, rats, and in further still another embodiment, mice. The animal's sex, age, body weight, and the like are not limited. Further, in a case of rats, rats at ages of about 1 month to about 24 months, weighing about 100 g to about 700 g, are preferably used, but the present invention is not limited thereto.

When the mammal is a non-human animal, it is preferable to use a genetically and microbiologically controlled population of animals. For example, it is genetically preferable to use an animal of an inbred strain or closed colony; in a case of rats, inbred rats such as Sprague-Dawley (SD), Wistar, and LEW can be mentioned as examples; in a case of mice, inbred mice such as BALB/c, C57BL/6, C3H/He, DBA/2, SJL, and CBA and closed colony mice such as DDY and ICR can be mentioned, but the present invention is not limited thereto. Although the animal may be a microbiologically conventional animal, it is more preferable to use an animal of specific pathogen free (SPF) or gnotobiotic grade from the viewpoint of elimination of the influence of infectious disease.

The method for administering the compound to an animal is not particularly limited as long as the compound is administered to the animal so as that a sufficient amount of the compound is delivered to target tissues (for example, urethra, bladder, uterus, and blood vessel). The dosage form is not particularly limited, but for example, the compound can be administered orally or parenterally (for example, intravenously, intramuscularly, intraperitoneally, intraarterially, subcutaneously, intradermally, intratracheally, and the like) in the form of a solid, a semi-solid, a liquid, an aerosol, or the like. The dose of the compound varies depending on the kind of compound, animal species, body weight, dosage form, and the like; for example, a dose required to expose the animal to the test compound at the highest concentration allowing the target cells to function for a given time or longer, as long as the animal can survive, and the like can be mentioned. In clinical studies, various doses are selected within a range established on the basis of the data obtained in pre-clinical studies.

As the method for measuring the urethral pressure, a conventional method is used. Specific examples thereof include the following methods using SD- or Wistar-based female rats.
(1) A catheter is mounted on the bladder dome to allow urine in the bladder to leak. The microchip transducer catheter (Milar Instruments Inc.) is inserted toward the bladder from the external urethral meatus and fixed such that the transducer portion is positioned around the portion at which the urethral pressure is maximized (10.0 mm to 15.0 mm from the external urethral meatus), and the urethral pressure is measured.
(2) The bladder dome is incised and a double lumen catheter (PE160 & PE50) (J. Urology, 162, 204-212 (1999)) is inserted toward the urethra from the bladder, and fixed such that the tip is positioned around the proximal urethral portion. Physiological saline is injected into the urethra at a predetermined flow rate through the external catheter and the urethral pressure is measured by a pressure transducer through the catheter inside.
(3) The bladder dome is incised and a catheter (PE100) with a barb at the tip is inserted toward the urethra from the bladder. The tip of the catheter is ligated and fixed in the bladder neck such that the tip is positioned around the proximal urethral portion. Further, the external urethral meatus is ligated. Physiological saline is injected into the urethra to a predetermined urethral pressure through a catheter. In addition, the urethral pressure is measured by a pressure transducer via a catheter.

In the present invention, a step of selecting a compound which specifically increases the urethral pressure can be further comprised. Here, the expression, specifically increases the urethral pressure, means that the compound increases the urethral pressure, but does not increase the internal pressure of other organs, for example, intravesical pressure, intrauterine pressure, blood pressure, or the like, or that the degree of an increase in the internal pressure of other tissues is small, relative to an increase in the urethral pressure.

For the measurement of the intravesical pressure, the intrauterine pressure, or the blood pressure, a method equivalent to the measurement of the urethral pressure is used. In addition, the blood pressure can also be measured using a cuff.

### 7. Method for Measuring Parameters of Urinary Incontinence Using Animals

As an animal used for the measurement of the parameters of urinary incontinence, the mammals as described above in the section of "6. Screening Method for Measuring Urethral Pressure" can be used. As the mammals, normal animals as well as disease model animals can be used.

The method for creating a model animal with stress urinary incontinence includes method for creating those prepared by known methods (1) to (4) below, but is not limited thereto.
(1) A method in which pelvic floor muscles or the surrounding tissues are destroyed by the progress of a balloon inserted into the vagina of an animal as a model indicating the weakening of a continence mechanism by the birth of a human (verginal distension method).
(2) A method in which the contraction of the urethral sphincter is reduced by damaging chemically or physically pudendal nerves governing the urethral sphincter.
(3) A method in which the contraction of the urethra is reduced by physically damaging nerves that control pelvic floor muscles, for example, nerves toward iliococcygeus muscles or pubococcygeus muscles.
(4) A method in which the urethra is treated with paraformaldehyde.

Examples of the parameters of urinary incontinence using mammals include (1) to (3) below, but are not limited thereto.
(1) The intravesical pressure at the time of urine leakage is determined when the animal is fixed vertically while physiological saline is injected into the bladder (modified leak point pressure).
(2) A change in the urethral pressure by transiently injecting physiological saline into the bladder to increase the intravesical pressure.
(3) The increased value of the urethral pressure, or the amount and frequency of urine leakage by an increase in the urethral pressure due to electrically stimulating the abdominal wall or inducing sneezing to increase the intravesical pressure.

In a patient, examples of the parameters of urinary incontinence include (1) to (4) below, but are not limited thereto.
(1) A pressure at a time of leakage which is a urethral resistance at a time of urine collection (LPP, leak point pressure).
(2) Presence or absence of urine leakage from the external urethral meatus when a patient coughs in the state where urine is sufficiently collected.
(3) An amount of urine leakage.
(4) Times of urination during a day of 24 hours and a voided volume each time.

Examples of the method for measuring the parameters of urinary incontinence using mammals include known methods (1) to (3) below, but are not limited thereto.
(1) A method for measuring the intravesical pressure at the time of urine leakage when an animal is fixed vertically while physiological saline is injected into the bladder (modified leak point pressure).
(2) A method for measuring the change in the urethral pressure by transiently injecting physiological saline into the bladder to increase the intravesical pressure.
(3) A method for measuring the increased value of the urethral pressure, or the amount and frequency of urine leakage by electrically stimulating the abdominal wall or inducing sneezing to increase the intravesical pressure.

Examples of the method for measuring the parameters of urinary incontinence in a patient include known methods (1) to (4) below, but are not limited thereto.
(1) A method for measuring the pressure at a time of leakage which is a urethral resistance at a time of urine collection (LPP, leak point pressure).
(2) A method for observing the amount and frequency of urine leakage from the external urethral meatus when a patient coughs in the state where urine is sufficiently collected (stress test).
(3) A method for determining the amount of urine leakage (pad test).
(4) A method for recording times of urination during a day of 24 hours and a voided volume each time.

Here, examples of the pad test include a 1-hour pad test for determining the incontinence amount by a difference between the pad weight before and after a 1-hour operation (walking, climbing stairs, coughing, and the like) causing stress urinary incontinence primarily after drinking 500 mL of water, a 24-hour pad test for determining the incontinence amount during 24 hours in day-to-day life, and the like.

By measuring the parameters of each urinary incontinence before and after the administration of the compound and comparing them, a compound having alleviated urinary incontinence can be selected as a compound for treating or preventing stress urinary incontinence or mixed urinary incontinence. It is believed that the compound having a melatonin receptor activating action improves the parameters of urinary incontinence in the measurement above, in a model animal or human patient with stress urinary incontinence, by contracting the urethra.

### 8. Measurement for Evaluating Therapeutic Effects for Patient

The therapeutic effects for a patient that undergoes a treatment of stress urinary incontinence or mixed urinary incontinence by administering a compound having a melatonin receptor activating action, using the parameters of urinary incontinence as described above in the section of "7. Method for Measuring Parameters of Urinary Incontinence Using Animals" can be evaluated. It is preferable to measure the amount of urine leakage as the parameters of urinary incontinence.

For example, the urine leaking from a patient before and after administration of a compound having a melatonin receptor activating action is collected as a sample and the amount of each urine leakage can be determined and comparison can be made therebetween. Further, when the amount of urine leakage is reduced, it can be evaluated that there is a practical effect of use. For the measurement of the amount of urine leakage, the above-described pad test can be used.

In one embodiment, the "therapeutic effects of stress urinary incontinence or mixed urinary incontinence" are a urethral pressure increasing action when the compound is administered to a subject, and in another embodiment, the therapeutic effects are the improvement of the parameters of urinary incontinence (for example, the above-described practical effect of use) when the compound is administered to a subject.

Furthermore, in one embodiment, the "compound increasing the urethral pressure" is a compound showing an action having a urethral pressure increasing action of 5 mmHg or more, when the compound is administered by the method shown in Example 4 as described later.

Here, when an active metabolite (ST-1059: purchased from CHEMIZON) of Midodrine (J. Urology, 118, 980-982 (1977)) which is an α₁) adrenoceptor agonist with which clinical effects on stress urinary incontinence have been identified is administered to a rat at a dose (0.01 mg/kg) estimated to correspond to the clinical dose using the same method as in Example 4 as described later (perfusion method), the urethral pressure increasing action was 4.9 ± 1.1 mmHg.

### 9. Use of the Inventive Screening Method

The screening method of the present invention is effective for screening of a compound for treating or preventing stress urinary incontinence or mixed urinary incontinence as well as for screening various drugs for other diseases by investigating the effects of the compound on the urethral contraction or urethral pressure. That is, the method can be effectively used for the purpose of, for example, excluding a compound that inhibits the urethral contraction or reduces the urethral pressure.

### 10. Method of Using Compound Comprised in Pharmaceutical Composition for Treating or Preventing Stress Urinary Incontinence or Mixed Urinary Incontinence

In the present invention, the compound comprised in the pharmaceutical composition of the present invention can be used in combination with various pharmaceutical compositions for treating or preventing the diseases for which the compound is considered to be effective. For the combined use, the respective pharmaceutical compositions may be administered simultaneously, or separately and continuously, or at a desired time interval. The preparations to be co-administered may be a combination preparation or may be prepared individually.

It can also be assumed that by using the active ingredients of the present invention in combination with other pharmaceutically active ingredients, the dose can be reduced, as compared with a case where other pharmaceutically active ingredients are administered alone. For example, in a case of the use in combination with a therapeutic agent for stress urinary incontinence, that is, duloxetine, the dose can be reduced, whereby reduction of side effects such as nausea, insomnia, dizziness, and suicidal tendencies can be promoted. By using the active ingredients of the present invention in combination with other pharmaceutically active ingredients having a different mechanism of action, the duration or increase of the therapeutic effect can be promoted or a synergetic effect can be obtained.

### [Examples]

Hereinbelow, the present invention will be described in detail with reference to Examples, but the Examples are simple exemplifications and do not limit the scope of the present invention in any case.

### Example 1

### Expression Analysis of MT1 Receptor Using Normal Tissues in Rats

### Experimental Method

Each of normal tissues of an SD rat having a size of about 0.1 g to 0.5 g with a body weight of 100 g to 500 g was collected, and then a total RNA was acquired using an RNeasy (registered trademark) Mini Kit (QIAGEN Inc.). cDNA was synthesized by SuperScript (registered trademark) III RT (Invitrogen Inc.), using a part of the acquired RNA. A PCR reaction was carried out using the synthesized cDNA as a template, a primer for an MT1 receptor gene consisting of a base sequence shown by SEQ ID NOS: 1 and 2, and SYBR (registered trademark) Green PCR Master Mix (Applied Biosystems), followed by analyzing with an Applied Biosystems (registered trademark) 7900HT Fast Real Time PCR system (Applied Biosystems). The expression amount of the gene was calculated using the number of copies of the MT1 receptor gene included in the reaction solution as a standard, based on rat genome-derived DNAs.

### Results

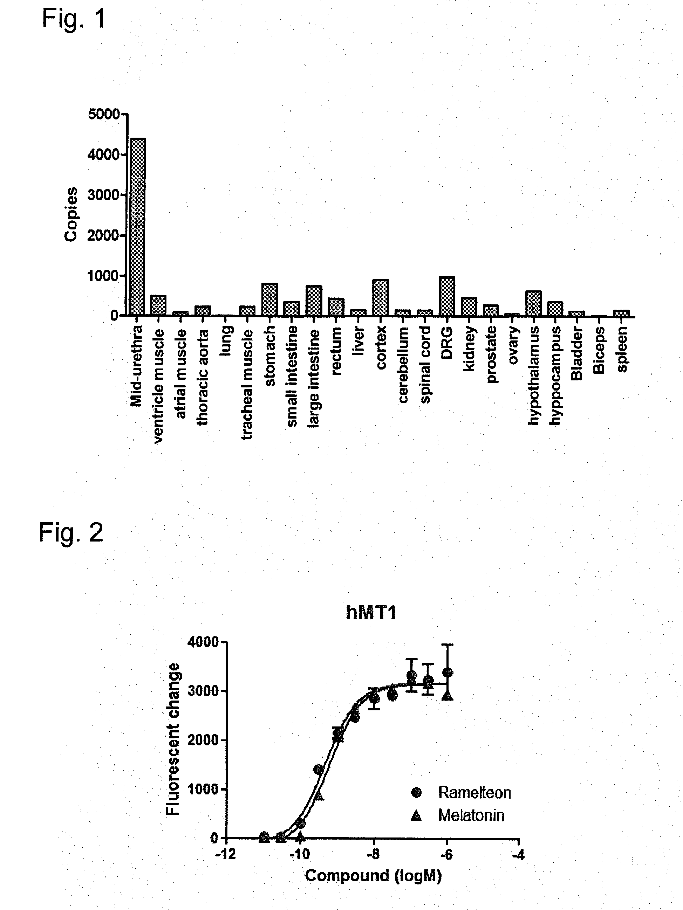

As shown in Fig. 1, it was confirmed that the MT1 receptor was highly expressed in the urethra (mid-urethra).

### Example 2

Test for Evaluating Activation of Human MT1 and MT2 Receptors of Compound Using Human MT1 and MT2 Receptor Expressing Cells

### Experimental Method

### (1) Isolation of Human-Derived MT1 and MT2 Receptors and Construction of Expression Vector

A human MT1 receptor gene (GenBank Access No.: NM_005958.3) and a human MT2 receptor gene (GenBank Access No.: NM_005959.3) were introduced into each expression vector pCDNA3.1/Zeo (Invitrogen Inc.).

### (2) Construction of Cells Stably Expressing Human MT1 and MT2 Receptors

The expression vectors for human MT1 and MT2 receptors were introduced into HEK293 cells, together with a G_{q/i} chimeric G protein expression vector, respectively. The incorporation was carried out in accordance with instructions, using a Lipofectoamine (registered trademark) 2000 Reagent (Invitrogen Inc.). Using Zeocin and hygromycin as a resistant agent, the cells were cultured for 15 days in the presence of 0.02 mg/mL and 0.05 mg/mL of the agents, respectively, thereby acquiring a drug-resistant clone.

### (3) Measurement of Intracellular Ca²⁺ Concentration by FLIPR (Registered Trademark)

The respective stably expressing cells were dispensed into 96 wells in a poly-D-lysine-coated plate (Falcon) the day before the experiment to give 40,000 cells/well, and cultured in a DMEM (Invitrogen Inc.) medium containing 10% FBS at 37°C and 5% CO₂ overnight. The medium was replaced with a loading buffer (a washing solution containing 4 µM Fluo-4AM (Dojindo Co.) (Hank's balanced salt solution (HBSS), 20 mM HEPES-NaOH, 2.5 mM probenecid)) and cultured at 37°C and 5% CO₂ for 1 hour. Thereafter, the cells were washed with a plate washer (ELx405, BIO-TEK Instrument Inc.) having a washing solution set therein, and set in an intracellular Ca concentration measuring system (FLIPR (registered trademark), Molecular Device Inc.). The compounds (melatonin, ramelteon, agomelatine, tasimelteon, and TIK-301) were each preliminarily dissolved in a washing solution and diluted to a final concentration of 10⁻⁵ M to 10⁻¹² M, set in an FLIPR (registered trademark) device together with cells, the cells were added to in the device, and a change in the intracellular Ca²⁺ concentration at that time was measured.

### Results

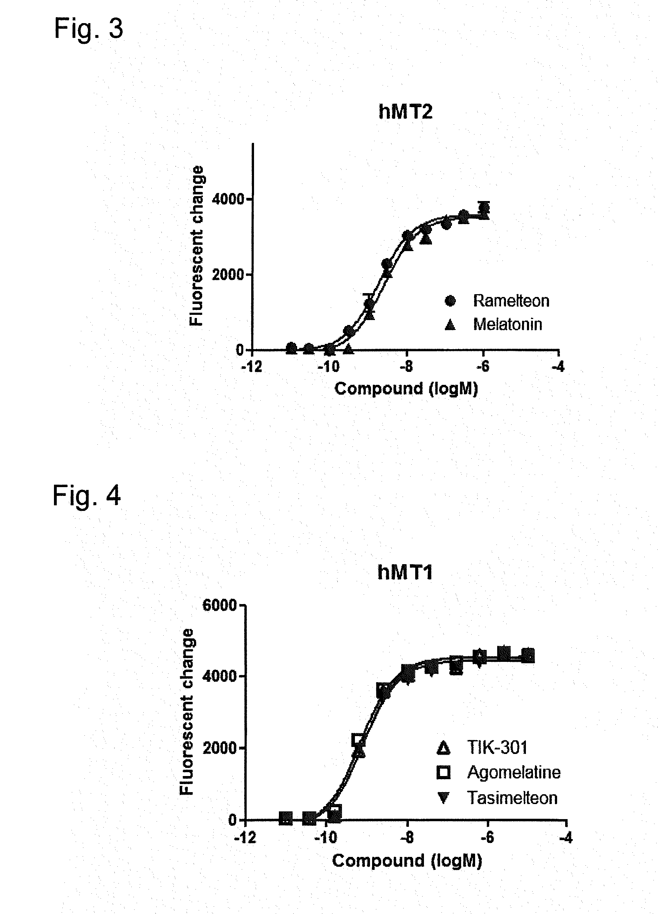

As shown in Figs. 2 to 5, melatonin, ramelteon, agomelatine, tasimelteon, and TIK-301 all increased the intracellular Ca²⁺ concentrations of the human MT1 and MT2 receptor expressing cells in a concentration-dependent manner. From above, it became clear that the compounds activate the human MT1 and MT2 receptors.

### Example 3

### Test for Evaluating Urethra Contracting Action of Compound Using Isolated Urethra

### Experimental Method

The urethra was isolated from an SD female rat having a body weight of 100 g to 500 g. The isolated urethra was incised vertically and then made into strip-shaped specimens having a width of about 3 mm. The specimens were suspended in a 10 mL tissue bus filled with a Krebs-Henseleit solution (pH 7.4) in the circular muscle direction. The Krebs-Henseleit solution was aerated at 95% O₂ and 5% CO₂, and warmed at 37°C. The resting tension was set to 0.5 g and the isometric contraction was recorded. After the resting tension was stabilized, a 10 µM phenylephrine solution was added thereto to cause contraction. The urethra specimens were washed and stabilized, and then the compounds (melatonin, ramelteon, agomelatine, tasimelteon, and TIK-301) were each cumulatively added to a final concentration of 10⁻⁵ M to 10⁻¹² M and the contraction response was measured.

### Results

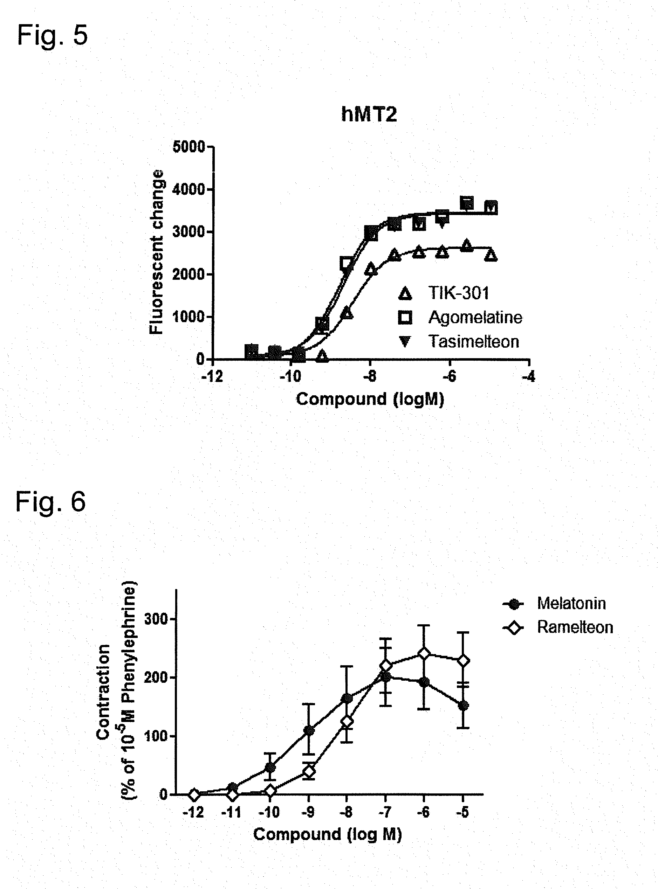

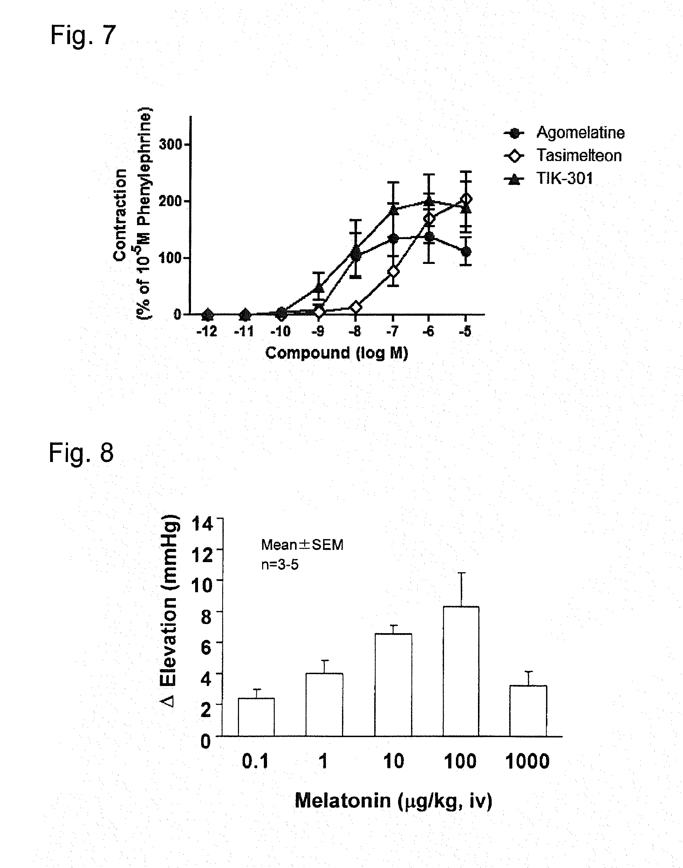

As shown in Figs. 6 and 7, melatonin, ramelteon, agomelatine, tasimelteon, and TIK-301 all contracted the isolated urethra. From above, it became clear that the compounds contract the urethral smooth muscle and/or the urethral sphincter.

### Example 4

### Test for Evaluating Effect of Compound on Urethral Pressure

### Experimental Method (Microchip Method)

An SD female rat was anesthetized with urethane and allowed to undergo laparotomy, and then a catheter was mounted in the bladder dome for urine leakage in the bladder. Further, a catheter was mounted in the femoral vein for the administration of a compound and a catheter was mounted in the carotid artery for the measurement of blood pressure described in Example 6 as described later. A microchip transducer catheter (Milar Instruments) was inserted toward the bladder from the external urethral meatus for the measurement of the urethral pressure, and fixed such that the transducer portion was positioned around the portion at which the urethral pressure was maximized (10.0 mm to 15.0 mm from the external urethral meatus). The catheter mounted in the carotid artery was attached to a pressure transducer and the blood pressure was measured. After the urethral pressure and the blood pressure were stabilized, compounds (melatonin and ramelteon) dissolved in physiological saline, or physiological saline containing 3% dimethyl sulfoxide (DMSO) and 2% Cremophor were intravenously administered at a dose of 0.1 µg/kg, 1 µg/kg, 10 µg/kg, 100 µg/kg, or 1000 µg/kg and a change in the urethral pressure was measured.

### Experimental Method (Perfusion Method)

An SD female rat was anesthetized with urethane and allowed to undergo laparotomy, the bladder dome was then incised, and a catheter having a barb at the tip was inserted toward the urethra from the bladder, and ligated and fixed such that the tip of the catheter was positioned around the proximal urethra. Physiological saline was injected at a predetermined flow rate into the urethra through the catheter and the urethral pressure was measured with a pressure transducer. After the urethral pressure was stabilized, compounds dissolved in physiological saline, or physiological saline containing 5% N,N-dimethylacetamide(DMA) and 0.5% Cremophor were intravenously administered at a dose of 1 µg/kg, 10 µg/kg, 100 µg/kg, or 1000 µg/kg and a change in the urethral pressure was measured.

### Results

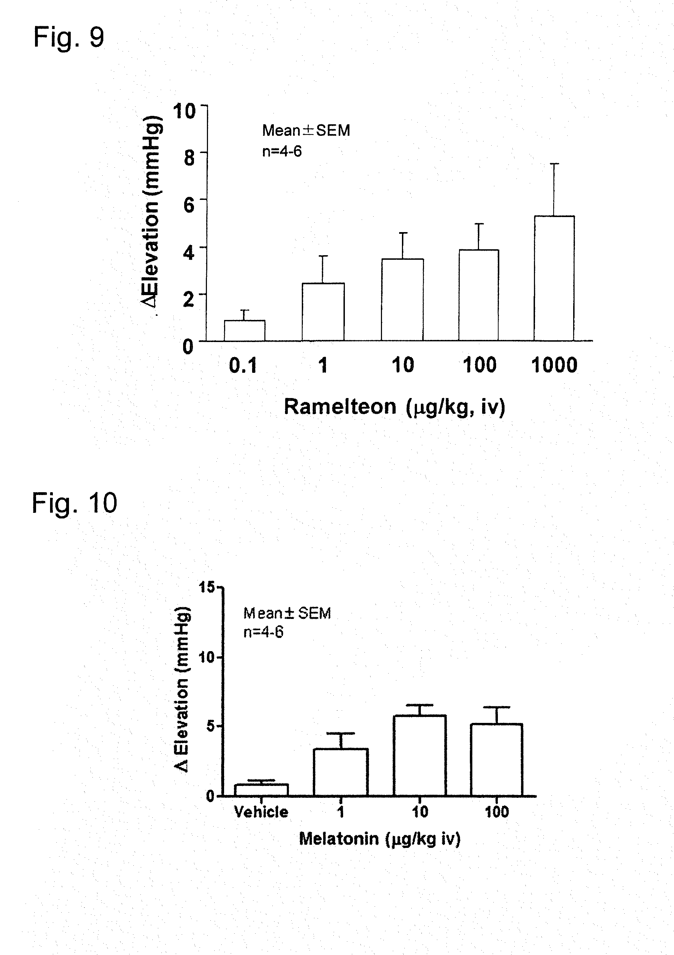

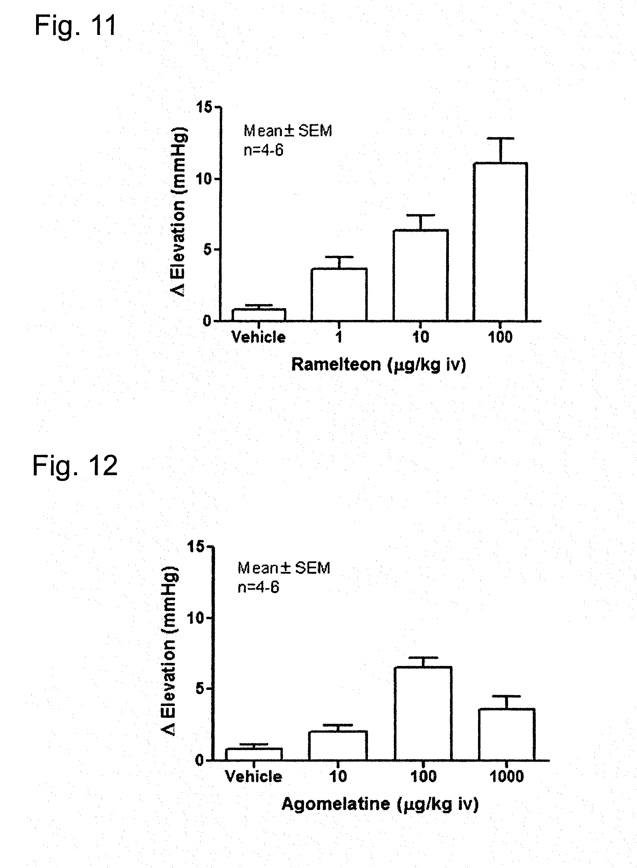

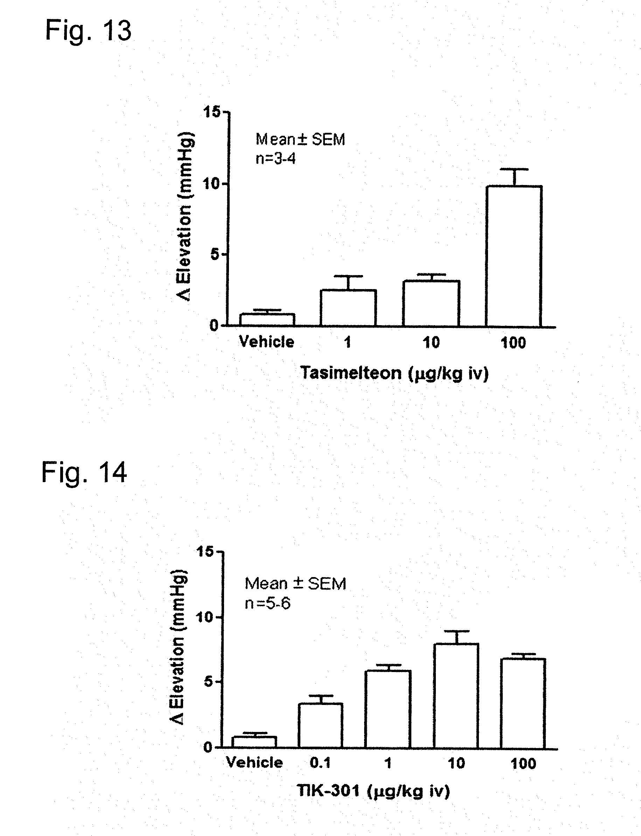

Figs. 8 and 9 show the results of the urethral pressure increasing actions of melatonin and ramelteon, measured by the microchip method. Figs. 10 to 14 show the results of the urethral pressure increasing actions of melatonin, ramelteon, agomelatine, tasimelteon, and TIK-301, measured by the perfusion method. As shown in Figs. 8 to 14, it was confirmed that melatonin, ramelteon, agomelatine, tasimelteon, and TIK-301 increased the urethral pressure. From above, it became clear that the compound plays a role in contracting the urethra even in vivo to increase the urethral pressure and thus maintain the continence.

### Example 5

### Test for Evaluation of Action of Compound as Action through MT1 and/or MT2 Receptor(s)

### Experimental Method

### (1) Measurement of Intracellular Ca²⁺ Concentration by FLIPR (Registered Trademark)

The respective stably expressing cells were dispensed into 96 wells in a poly-D-lysine-coated plate (Falcon) the day before the experiment to give 40,000 cells/well, and cultured in a DMEM (Invitrogen Inc.) medium containing 10% FBS at 37°C and 5% CO₂ overnight. The medium was replaced with a loading buffer (a washing solution containing 4 µM Fluo-4AM (Dojindo Co.) (Hank's balanced salt solution (HBSS), 20 mM HEPES-NaOH, 2.5 mM probenecid)) and cultured at 37°C and 5% CO₂ for 1 hour. Thereafter, the cells were washed with a plate washer (ELx405, BIO-TEK Instrument Inc.) having a washing solution set therein, and set in an intracellular Ca concentration measuring system (FLIPR (registered trademark), Molecular Device Inc.). Luzindole was preliminarily dissolved in a washing solution, diluted to a final concentration of 10⁻⁵ M to 10⁻⁷ M, and left to stand at room temperature for 5 minutes. Ramelteon was preliminarily dissolved in a washing solution and diluted to a final concentration of 10⁻⁵ M to 10⁻¹¹ M, the resultant was set in an FLIPR (registered trademark) device together with the cells after leaving to stand for 5 minutes after the addition of luzindole, the cells were added to the device, and a change in the intracellular Ca²⁺ concentration at that time was measured.

### (2) Measurement of Urethra Contracting Action of Compound Using Isolated Urethra

The urethra was isolated from an SD female rat having a body weight of 100 g to 500 g. The isolated urethra was incised vertically and then made into strip-shaped specimens having a width of about 3 mm. The specimens were suspended in a 10 mL tissue bus filled with a Krebs-Henseleit solution (pH 7.4) in the circular muscle direction or the longitudinal muscle direction. The Krebs-Henseleit solution was aerated at 95% O₂ and 5% CO₂, and warmed at 37°C. The resting tension was set to 0.5 g and the isometric contraction was recorded. After the resting tension was stabilized, a 10 µM phenylephrine solution was added thereto to cause contraction. After washing and stabilizing, the compound (ramelteon) was cumulatively added to a final concentration of 10⁻⁵ M to 10⁻¹² M and the contraction response was measured. After washing and stabilizing, luzindole was added, after 20 minutes, ramelteon was cumulatively added again, and the contraction response was measured. When the contraction by 1 µM ramelteon at the first time was taken as 100%, the contraction rate by ramelteon at the second time was used for the evaluation.

### Results

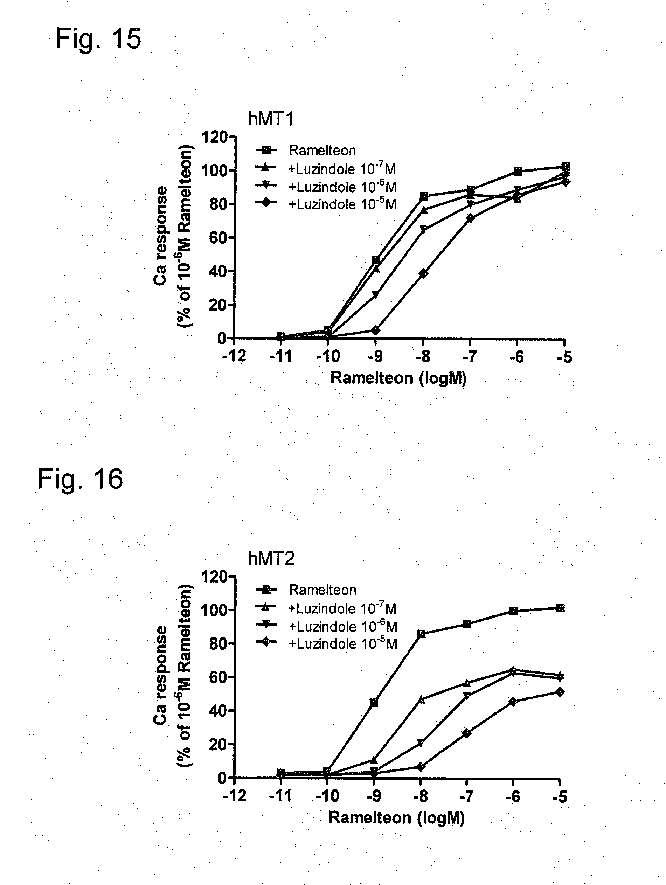

As seen in Figs. 15 and 16, the increases in the intracellular Ca²⁺ concentration by ramelteon in the MT1 and MT2 receptor expressing cells were each inhibited. As seen in Fig. 15, in the MT1 receptor expressing cells, the intracellular Ca²⁺ concentration increasing actions at a final concentration of ramelteon of 10⁻⁵ M with the addition of 10⁻⁷ M luzindole, 10⁻⁶ M luzindole, and 10⁻⁵ M luzindole were 100%, 97%, and 94%, respectively. On the other hand, as shown in Fig. 16, in the MT2 receptor expressing cells, the intracellular Ca²⁺ concentration increasing actions at a final concentration of ramelteon of 10⁻⁵ M with the addition of 10⁻⁷ M luzindole, 10⁻⁶ M luzindole, and 10⁻⁵ M luzindole were 62%, 60%, and 52%, respectively.

In addition, the inhibiting action by luzindole was recognized to be stronger in the MT2 receptor expressing cells than in the MT1 receptor expressing cells, and it has been reported that luzindole has an inhibiting action on the MT1 and MT2 receptors and it has also been reported that the inhibiting action is stronger on the MT2 receptor.

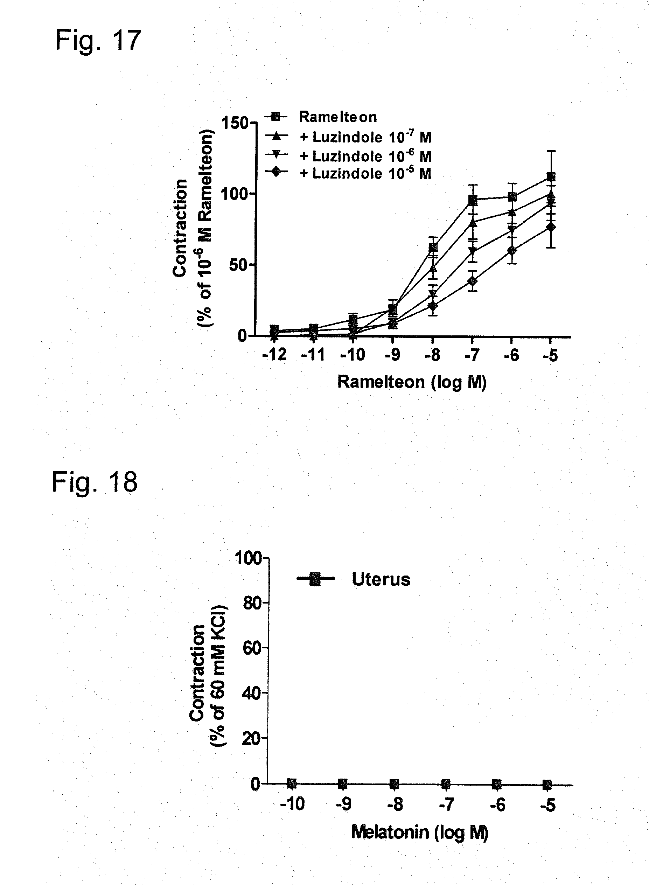

On the other hand, as shown in Fig. 17, the isolated urethra contracting action by ramelteon was inhibited in a concentration-dependent manner by a treatment with luzindole. The isolated urethra contracting actions at a final concentration of ramelteon of 10⁻⁵ M with the addition of 10⁻⁷ M luzindole, 10⁻⁶ M luzindole, and 10⁻⁵ M luzindole were 101 %, 94%, and 78%, respectively.

The luzindole substantially did not exhibit an inhibiting action on the MT1 receptor at a final concentration of ramelteon of 10⁻⁵ M, but it exhibited an inhibiting action at 40% to 50% on the MT2 receptor. On the other hand, for the isolated urethra contracting action, luzindole substantially did not exhibit an inhibiting action at a final concentration of ramelteon of 10⁻⁵ M. From this point of view, it has been suggested that the urethral smooth muscle and/or urethral sphincter contracting action by ramelteon is based on the MT1 receptor activating action.

### Example 6

### Test for Evaluating Effect of Compound on Isolated Bladder, Isolated Uterus, and Blood Pressure

### Experimental Method

(1) The bladder and uterus were isolated from an SD female rat having a body weight of 100 g to 500 g. For the isolated bladder, the parts below the parietal region and the triangular portion were resected to make only the body portion into a ring state, and further, one side of the tissue in the ring state was cut to a band-shaped tissue. The bladder in the band shape was isolated parallel to the median axis of the bladder to prepare strip-shaped specimens having a length of about 10 mm and a width of about 3 mm. The specimens were suspended in a 10 mL tissue bus filled with a Krebs-Henseleit solution (pH 7.4) at a resting tension of 0.5 g and the isometric contraction was recorded. For the isolated uterus, the edge portion of the uterus was cut and incised vertically, whereby a strip-shaped specimen having a width of about 6 mm was then prepared. The specimens were suspended in a 10 mL tissue bus filled with a Krebs-Henseleit solution (pH 7.4) at a resting tension of 0.5 g and the isometric contraction was recorded. The Krebs-Henseleit solution was aerated at 95% O₂ and 5% CO₂, and warmed at 37°C. For the bladder specimen, after the resting tension was stabilized, a 60 mM KCl solution or a 10 µM carbachol solution was added thereto to cause contraction. The specimens were washed and stabilized, and then the compounds were cumulatively administered. For the uterus specimen, stable rhythmic contraction was confirmed and then the compound (melatonin or ramelteon) was cumulatively added to the specimen to a final concentration of 10⁻⁵ M to 10⁻¹¹ M. After investigating the action of the compound, a 60 mM KCl solution was added thereto to cause contraction.
(2) In the experiment for measuring the urethral pressure in Example 4, the blood pressure was measured simultaneously. After the urethral pressure and the blood pressure were stabilized, the compound was administered and a change in the blood pressure was measured.

### Results

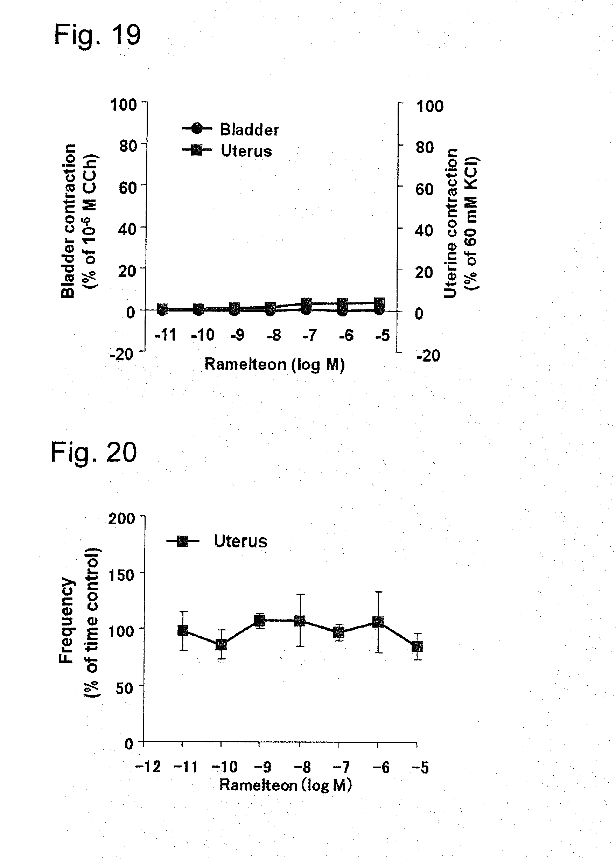

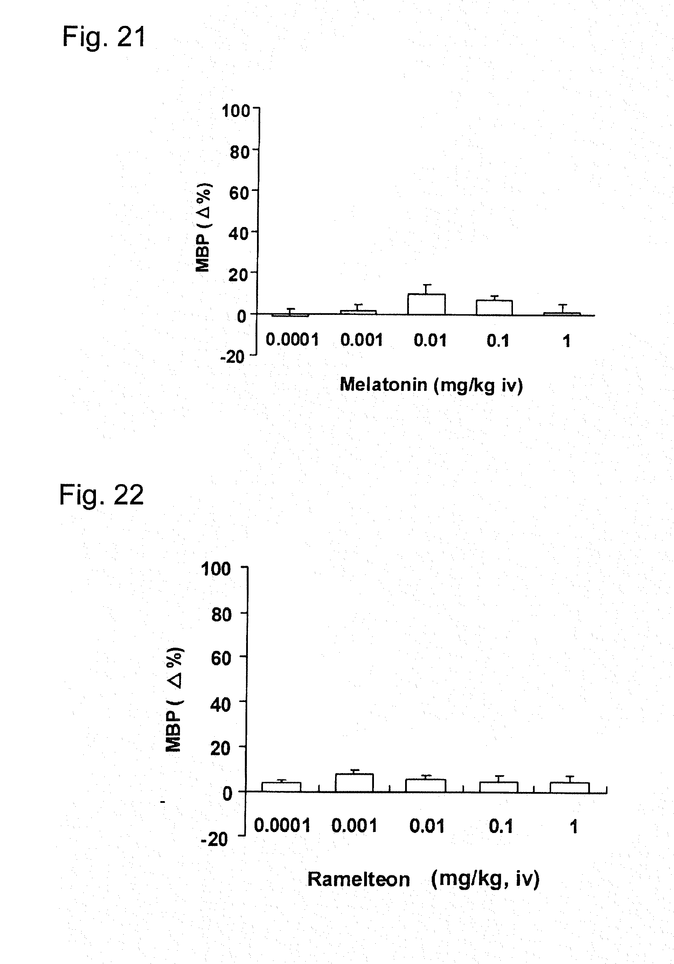

As shown in Figs. 18 to 20, melatonin and ramelteon did not exhibit a contraction action on the isolated bladder and the isolated uterus. Further, ramelteon did not have an effect on the rhythmic contraction of the isolated uterus. In addition, as shown in Figs. 21 and 22, melatonin and ramelteon did not increase the blood pressure at a dose for increasing the urethral pressure. From above, it became clear that melatonin and ramelteon play a role in specifically contracting the urethra even in vivo to increase the urethral pressure and thus maintain the continence.

From above, it became clear that a compound having a melatonin receptor activating action plays a role in specifically contracting the urethra to maintain the continence. Further, as shown in Example 5, it has been suggested that the urethra contracting action by ramelteon is an action based on the MT1 receptor activating action. Accordingly, it was found that a compound having a melatonin receptor activating action is useful as a pharmaceutical composition for treating or preventing stress urinary incontinence or mixed urinary incontinence, and the method and the screening tool of the present invention are useful as a method for screening compounds for treating or preventing stress urinary incontinence or mixed urinary incontinence.

### Preparation Example

(1) Ramelteon 1.0 g
(2) Lactose 60.0 g
(3) Corn starch 35.0 g
(4) Gelatin 3.0 g
(5) Magnesium stearate 2.0 g

A mixture of 1.0 g of ramelteon, 60.0 g of lactose, and 35.0 g of corn starch was granulated using a 10% by weight of gelatin aqueous solution (3.0 g in terms of gelatin) through a 1 mm mesh screen, then dried at 40°C, and screened again. The obtained granules were mixed with 2.0 g of magnesium stearate and compressed. The obtained core tablets were sugar-coated with an aqueous suspension containing cane sugar, titanium dioxide, talc, and arabic gum. The coated tablets were polished with beeswax to obtain 1000 coated tablets.

### Industrial Applicability

As described above, the compound of the present invention can be used as a pharmaceutical composition for treating or preventing stress urinary incontinence or mixed urinary incontinence. In addition, the method and screening tool of the present invention can be used to screen a compound for treating or preventing stress urinary incontinence or mixed urinary incontinence.

### [Sequence List Free Text]

SEQ ID NO: 1: PCR primer (for an MT1 receptor)
SEQ ID NO: 2: PCR primer (for an MT1 receptor)

## Claims

1. A method for selecting a compound for treating or preventing stress urinary incontinence or mixed urinary incontinence, comprising evaluating a melatonin receptor activating action.

2. A method for selecting a compound for treating or preventing stress urinary incontinence or mixed urinary incontinence, comprising:
a step of bringing the compound into contact with a melatonin receptor expressing cell;
a step of measuring a melatonin receptor activation; and
a step of selecting a compound that activates a melatonin receptor.

3. The method according to claim 2, further comprising a step of confirming that the selected compound has a therapeutic effect on stress urinary incontinence or mixed urinary incontinence.

4. The method according to claim 2, further comprising:
a step of bringing the selected compound into contact with the isolated urethra;
a step of measuring the contraction of the isolated urethra; and
a step of selecting a compound which contracts the isolated urethra.

5. The method according to claim 3, wherein the step of confirming the therapeutic effect comprises:
a step of administering the selected compound to a subject;
a step of measuring the urethral pressure of the subject; and
a step of selecting a compound increasing the urethral pressure.

6. The method according to claim 3, wherein the step of confirming the therapeutic effect is a step of measuring the parameters of urinary incontinence before and after administering the selected compound to a subject.

7. The method according to any one of claims 1 to 6, wherein the melatonin receptor is an MT1 receptor.

8. A screening tool used in a method for selecting a compound for treating or preventing stress urinary incontinence or mixed urinary incontinence, comprising a melatonin receptor polypeptide or a melatonin receptor expressing cell.

9. Use of a melatonin receptor polypeptide or a melatonin receptor expressing cell for the manufacture of a screening tool used in a method for selecting a compound for treating or preventing stress urinary incontinence or mixed urinary incontinence.

10. Use of a melatonin receptor polypeptide or a melatonin receptor expressing cell as a screening tool used in a method for selecting a compound for treating or preventing stress urinary incontinence or mixed urinary incontinence.

11. A pharmaceutical composition for treating or preventing stress urinary incontinence or mixed urinary incontinence, comprising a compound having a melatonin receptor activating action as an active ingredient.

12. The pharmaceutical composition according to claim 11, wherein the melatonin receptor is an MT1 receptor.

13. The pharmaceutical composition according to claim 11 or 12, wherein the compound having a melatonin receptor activating action is a compound selected from the group consisting of melatonin, ramelteon, agomelatine, tasimelteon, and TIK-301 and a pharmaceutically acceptable salt thereof.

14. A method for treating or preventing stress urinary incontinence or mixed urinary incontinence, comprising administering an effective amount of a compound having a melatonin receptor activating action to a subject.

15. The method according to claim 14, wherein the melatonin receptor is an MT1 receptor.

16. The method according to claim 14 or 15, wherein the compound having a melatonin receptor activating action is a compound selected from the group consisting of melatonin, ramelteon), agomelatine, tasimelteon, and TIK-301 and a pharmaceutically acceptable salt thereof.

17. Use of a compound having a melatonin receptor activating action for the manufacture of a pharmaceutical composition for treating or preventing stress urinary incontinence or mixed urinary incontinence.

18. The use of the compound according to claim 17, wherein the melatonin receptor is an MT1 receptor.

19. The use of the compound according to claim 17 or 18, wherein the compound having a melatonin receptor activating action is a compound selected from the group consisting of melatonin, ramelteon, agomelatine, tasimelteon, and TIK-301 and a pharmaceutically acceptable salt thereof.

20. Use of a compound having a melatonin receptor activating action for the treatment of stress urinary incontinence or mixed urinary incontinence.

21. The use of the compound according to claim 20, wherein the melatonin receptor is an MT1 receptor.

22. The use of the compound according to claim 20 or 21, wherein the compound having a melatonin receptor activating action is a compound selected from melatonin, ramelteon, agomelatine, tasimelteon, and TIK-301 and a pharmaceutically acceptable salt thereof.

23. A compound having a melatonin receptor activating action for the treatment of stress urinary incontinence or mixed urinary incontinence.

24. The compound according to claim 23, wherein the melatonin receptor is an MT1 receptor.

25. The compound according to claim 23 or 24, wherein the compound having a melatonin receptor activating action is a compound selected from the group consisting of melatonin, ramelteon, agomelatine, tasimelteon, and TIK-301 and a pharmaceutically acceptable salt thereof.


SEQUENCE LISTING

<110> Astellas Pharma Inc.

<120> AGENT FOR THE TREAMENT OR PREVENTION OF STRESS URINARY INCONTINENCE OR
MIXED URINARY INCONTINENCE AND METHOD OF SCREENING OF THE SAME

<130> A13024A00

<160> 2

<210> 1
<211> 21
<212> DNA
<213> Artificial
<220>
<223> Description of Artificial Sequence:an artificially synthesized primer
sequence
<220>
<223> Inventor: Toru, Watanabe; Keiko, Kawabata; Yuka, Watanabe; Hironori,
Yuyama;
\201@\201@\201@Inventor: Noriyuki, Masuda; Edward, C., Burgard; Karl, Bruce,
Thor
<400> 1
actcatagccatcatgcccaa

<210> 2
<211> 21
<212> DNA
<213> Artificial
<220>
<223> Description of Artificial Sequence:an artificially synthesized primer
sequence
<220>
<223> Inventor: Toru, Watanabe; Keiko, Kawabata; Yuka, Watanabe; Hironori,
Yuyama;
\201@\201@\201@Inventor: Noriyuki, Masuda; Edward, C., Burgard; Karl, Bruce,
Thor
<400> 2
gggtcgtactggagagttccg